# EUROPEAN PATENT SPECIFICATION

(11) **EP 2 962 641 B1**
(45) Date of publication and mention of the grant of the patent: **28.06.2017**
(21) Application number: 13876111.9
(22) Date of filing: 09.08.2013
(51) Int. Cl.: A61B 6/00, A61B 6/03, G01T 1/29

(54) **RING TOPOLOGICAL STRUCTURE OF PET IMAGING SYSTEM AND IMPLEMENTATION METHOD THEREOF**
STRUKTUR MIT RINGTOPOLOGIE FÜR EIN PET-BILDGEBUNGSSYSTEM UND IMPLEMENTIERUNGSVERFAHREN DAFÜR
STRUCTURE TOPOLOGIQUE ANNULAIRE D'UN SYSTÈME D'IMAGERIE TEP ET PROCÉDÉ DE MISE EN OEUVRE ASSOCIÉ

(30) Priority: 01.03.2013 CN 201310064672
(43) Date of publication of application: 06.01.2016
(73) Proprietor: Jiangsu Sinoways (Zhonghui) Medical Technology Co., Ltd., Yangzhou, Jiangsu 225200 (CN)
(72) Inventor: WU, Heyu, Yangzhou Jiangsu 225200 (CN); ZHANG, Hui, Yangzhou Jiangsu 225200 (CN); WANG, Yi, Yangzhou Jiangsu 225200 (CN); JU, Xiaoping, Yangzhou Jiangsu 225200 (CN)
(74) Representative: ProI European Patent Attorneys
(86) International application number: PCT/CN2013/081183
(87) International publication number: WO 2014/131267

(56) References cited:
- WO-A1-2009/060348
- WO-A2-2010/048626
- WO-A2-2010/067220
- CN-A- 101 855 564
- CN-A- 102 302 372
- CN-A- 102 631 212
- CN-A- 103 099 639
- US-A- 5 241 181
- US-A1- 2004 195 512

## Description

### BACKGROUND

### Technical Field

The present invention relates to a basic topological structure of a PET, PET/CT, and PET/MR medical imaging system, and an implementation method of the basic structure.

### Related Art

PET is an instrument for nuclear medical functional imaging, which can be used for fast diagnosis of a cancer cell mass, particularly early-stage cancers, and is a good tool for diagnosis and treatment of tumors, cardiovascular diseases and neurologic diseases and is thus clinically used rapidly. As a large-scale radioactive surgery system, the PET has ten thousands or hundred thousands of measuring detector units for imaging. By reducing the quantity of designed detector channels to thousands, an imaging effect is achieved: in several minutes, millions of coincidence events are at least obtained, and only a distributed collecting mode can be adopted to judge and discard millions of non-coinciding single events. An example of known PET systems is given in WO-2009/060348, which shows a PET system with a ring of detectors and a separate coincidence detection electronics module. A star structure is a collecting mode usually adopted by the current PET system. After being amplified and formed, the signal of each detector is converted to digital information by independent ADs, data of a plurality of ADs are converged into one concentrated processing unit (front-end) for processing so as to judge useful full-energy peak events, and the position of rays at the minimal imaging unit is analyzed; all processing unit events are converged into one central processing unit, namely the conformation processing unit, and coincidence events are judged and output in a concentrated manner.

The star topological structure is a natural logic result, and distributed information is processed and output in a concentrated manner. But, when design of such a structure is completed, an imaging system is unique and fixed, and is a system which cannot be changed on hardware. Branch quantity for receiving information at the central processing unit and branch information capacity, namely the quantity of detector units for processing is fixed, and any small modification to the hardware results in the brand new design of the whole PET system. When development of the high performance PET system faces the high hardware IO, the central processing unit also faces challenges of great processing capacity and data bandwidth.

### SUMMARY

The present invention aims to provide an implementation method of a topological structure of a PET imaging system, which can reduce processing workload of coincidence modules, increase IO channel quantity of hardware and randomly increase or reduce processing capacity of ray detectors of the system, so as to conveniently produce three-ring, four-ring, five-ring and six-ring systems.

Event coincidence judging of the PET system is completed by a series of distributed conformation modules, the coincidence modules are serially connected into a ring structure, and each conformation module is a node module on a link; and at least one node module is filtered out from the ring structure to take out a conforming event.

A specific method includes: electronic signals of a plurality of groups of detectors are converged to one event processing module, forming, digitalization and digital analysis of signals are completed at the event processing module, and a full energy peak event is selected; information of a plurality of event processing modules is output and converged to one node module, and the event coincidence judging is performed in the node module; a complex system forms star branches by adopting a plurality of event processing modules and the coincidence modules, and respective branched coincidence modules are cascaded to form a ring star structure, and functions of the processing modules and functions of the coincidence modules in a simple imaging system are combined together to form the node modules, which are cascaded to form a single ring structure.

A topological structure of the present invention includes event processing modules used for receiving and processing signals from a plurality of ray detectors, an event coincidence detection node module for receiving events from the plurality of event processing modules, and at least one coincidence event filter node module; each of the node modules and the filter node module are sequentially serially connected by a bus or point-to-point data transmission to form a closed ring structure, and data information is generated on the detectors, is firstly converged to the event processing modules, is further converged to the node modules to enter the ring structure, and circularly flows in the ring structure.
each node module caches the information converged on the node module for coincidence detection, and directly issues the information of the node module to the next node module, so the information of a node flows in the ring structure;
each node module judges and deletes information from the node module or the outdated information from the last node module, forbids the continuous transmitting of the deleted information and balances and reduces data volume continuously entering the ring;
each node module performs time judgment on the event information from the last node module and event information of the node module, judges two events simultaneously occurring in a coincidence time window as a coincidence event and then marks the information of the coincidence event; each node module sends the information of the coincidence event or the information of an non-outdated single event from the last node module to the next node module, so the coincidence event is transmitted to a coincidence event filter node and the single event has bigger chance to be subject to coincidence judging together with the events on more nodes; and
the filter node detects the information of a coincidence mark event, forbids the detected information of the coincidence mark event from continuously transmitting to the next node module, and sends the information of the coincidence event to a computer for data collection.

The work principle of the present invention is as follows: signals of respective ray detectors are converged to the event processing modules (front-end function modules) through AD conversion, and full-energy peak judgment and analysis on a detected crystal position are realized after processing of the event processing modules; event information of a plurality of event processing modules are converged to one coincidence node module, a signal transmission loop is formed by serial connection of lots of node modules, and coincidence judging of events is finished on the loop; the nodes send conformed new information to the next node module, namely, the coincidence data are circulated in the loop till reaching at the filter node module having detected coincidence mark event information, and transmitting the detected coincidence mark event information to a connected calculation center, the filtered coincidence event information is further transmitted to a detection and control function module of the PET system under control of the calculation center; events screened by the event processing modules are single events, a copy is cached when the single events are converged to the nodes and is kept till coincidence is detected or outdating is detected, and the other copy is injected into the loop for circulation till coincidence is detected or outdating is detected.

The present invention realizes a loop structure of a central processing unit, concentrated tasks are distributed to respective node modules, a hardware structure and a software (FPGA) structure of each node are same in function, tasks become simple and effective, 10 quantity of each unit is reduced, use efficiency of a data transmission link is improved, but total 10 quantity of the system is increased and a processing capacity is greatly improved. More importantly, according to the PET system with such a ring structure, by increasing and reducing node quantity and node processing quantity, block building type production of a PET system is achieved without changing hardware electronics and FPGA program in the hardware, namely without changing system design, and production of clinical and research PET from high class to low class is achieved according to user needs. By using this structure, calculation volume and 10 load of the coincidence processing units are greatly reduced, switching between the production of a simple system and the production of a complex system is implemented, and research and development efficiency is greatly improved. The production of the PET system presents a block building effect, three-ring, four-ring, five-ring and six-ring systems can be conveniently produced, switching between high and low resolution and production switching of a small system of a small animal and a large system of a human body is implementing by increasing or reducing node quantity and changing size of crystal in a detector.

### BRIEF DESCRIPTION OF THE DRAWINGS

FIG. 1 is a ring plus star topological structure and data flow schematic diagram when the present invention is used in a large system of a human body; and
FIG. 2 is a ring topological structure and data flow schematic diagram when the present invention is used in a small system of a small animal.

### DETAILED DESCRIPTION

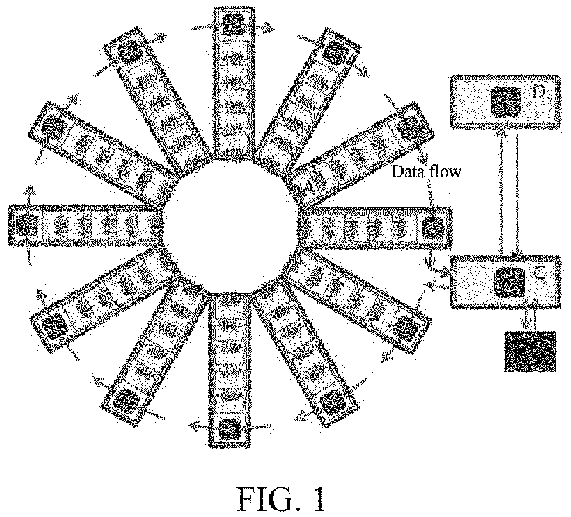

As shown in FIG. 1, A are pluggable event processing modules (front-end function modules) for receiving and processing signals form a plurality of ray detectors, B are coincidence node modules, C is a filter node module of a coincidence event in the ring structure, and D is a detection and control function module of the PET system. PC is a data collection computer.

Respective processing units A can be (but not limited to) a case pluggable manner, respective node modules B can be arranged on a motherboard of a case, the node modules B are linked in a variable socket manner, and the modules B can automatically identify a plugboard of the case.

Respective node modules B and the filter node module C are sequentially serially connected by a bus or point-to-point data transmission to form a closed ring structure, that is to say, respective cases are serially connected by a data line.

Each node module B caches information of the node module for coincidence detection, and directly issues the information of the node module to the next node module.

Each node module judges and deletes the information from the node module or outdated information from the last node module and forbids the continuous transmitting of the deleted information.

Each node module performs time judgment on the information from the last node module and the information of the node module, judges a coincidence event in a coincidence time window and then marks the information of the coincidence event.

Each node module sends the information of the conforming event or the information of a non-outdated single event from the last node module to the next node module;

The filter node module C detects the information of a coincidence mark event, sends the information of the detected coincidence event to a connected calculation center PC, and forbids the detected information of the coincidence mark event from continuously transmitting to the next node module.

Under control of the calculation center PC, the information of the detected coincidence mark event is transmitted to the detection and control function module D of the PET system.

All above function modules and node modules generally refer to FPGAs of hardware so as to obtain a high speed timely processing capacity.

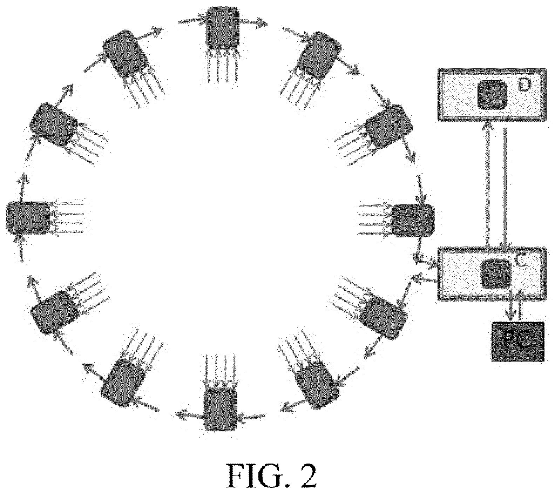

As shown in FIG. 2, the shown ring structure is same as the star plus ring structure as shown in FIG. 1, respective node modules B and the filter node module C are sequentially serially connected by a bus or point-to-point data transmission to form a closed ring structure, respective node modules and the filter node module have the completely same event coincidence judgment and filter and event information transmittance and outdating deleting functions. The system is simple, so quantity of the detectors is small, signals of the detectors are directly connected to the node modules, which finish the function of the last level of event analysis modules, thus simplifying the system structure.

## Claims

1. An implementation method of a ring topological structure of a PET imaging system, wherein coincidence event judging of the PET system is completed by a series of distributed coincidence modules serially connected into the ring structure, and each coincidence module is a node module on a link; and at least one node module on the ring structure filters out a coincidence event.

2. The method according to claim 1, wherein electronic signals of a plurality of groups of detectors are converged to one event processing module, forming, digitalization and digital analysis of a signal are completed at the event processing module, and a full energy peak event is selected; information of a plurality of event processing modules is output and converged to one node module, and the coincidence event judging is performed in the node module.

3. The method according to claim 2, wherein a complex PET imaging system comprises a plurality of coincidence modules formed into the ring structure wherein each coincidence module is linked to a branch formed by a plurality of event processing modules.

4. The method according to claim 2, wherein a simple PET imaging system comprises a plurality of node modules formed into the ring structure wherein each node structure performs the function of an event processing module and the function of the a coincidence module.

5. A ring topological structure of a PET imaging system, comprising:
event processing modules for receiving and processing signals from a plurality of ray detectors,
an event coincidence detection node module for receiving events from the plurality of event processing modules, and
at least one coincidence event filter node module;
wherein
each of the node modules and the filter node module are sequentially serially connected by a bus or point-to-point data transmission to form a closed ring structure;
each node module is configured to cache information of the node module itself for facilitating coincidence detection, and is configured to directly transmit the information of the node module to the next node module;
each node module is configured to judge and delete the information from the node module itself or outdated information transmitted from the previous node module and forbid further transmission of the deleted information;
each node module is configured to perform time judgment on the information received from the previous node module and the information of the node module itself, judge a coincidence event in a coincidence time window and then mark the information of the coincidence event;
each node module is configured to send the information of the coincidence event or the information of non-outdated single event from the previous node module to the next node module; and
the filter node module is configured to detect the information of a coincidence mark event, forbid the detected information of the coincidence mark event from being further transmitted to the next node module, and send the information of the detected coincidence event to a computer for data collection.

## Patentansprüche

1. Verfahren zum Verwirklichen einer ringförmigen topologischen Struktur für ein PET-Bildgebungssystem, **dadurch gekennzeichnet, dass** die Feststellung über die Konformität eines Ereignisses eines PET-Systems durch eine Reihe von verteilten Konformitätsmodulen erfolgt, welche Konformitätsmodule in Reihe geschaltet werden, um eine ringförmige Struktur zu bilden, wobei jedes der Konformitätsmodule ein Knotenmodul auf einem Link darstellt, wobei die Entnahme eines konformen Ereignisses durch mindestens ein Ausfilterungs-Knotenmodul in der ringförmigen Struktur erfolgt.

2. Verfahren nach Anspruch 1, **dadurch gekennzeichnet, dass** elektronische Signale mehrerer Gruppen von Detektoren zu einem Ereignisbehandlungsmodul zusammengebracht werden, in dem die Formung, Digitalisierung und digitale Analyse der Signale sowie das Wählen eines Vollenergiespitze-Ereignisses erfolgen, wobei die Information mehrerer Ereignisbehandlungsmodule zu einem Knotenmodul ausgegeben und zusammengebracht wird, in dem die Feststellung über die Konformität des Ereignisses erfolgt.

3. Verfahren nach Anspruch 2, **dadurch gekennzeichnet, dass** ein PET-Bildgebungssystem ferner mehrere Konformitätsmodule umfasst, die eine ringförmige Struktur bilden, wobei die Konformitätsmodule der jeweiligen Zweige über mehrere Ereignisbehandlungsmodule einen sternförmigen Zweig bilden.

4. Verfahren nach Anspruch 2, **dadurch gekennzeichnet, dass** ein PET-Bildgebungssystem ferner mehrere Knotenmodule umfasst, die eine ringförmige Struktur bilden, wobei jedes der Knotenmodule die Funktion des Ereignisbehandlungsmoduls und des Konformitätsmoduls erfüllt.

5. Ringförmige topologische Struktur für ein PET-Bildgebungssystem, **dadurch gekennzeichnet, dass** sie ein zum Empfangen und Behandeln eines von einem Multikanal-Lichtstrahldetektor stammenden Signals dienendes Ereignisbehandlungsmodul, ein zum Empfangen eines Multikanal-Ereignisbehandlungsmoduls dienendes Knotenmodul zur Überprüfung der Ereigniskonformität und zumindest ein Knotenmodul zum Ausfiltern eines konformen Ereignisses umfasst, wobei die jeweiligen Knotenmodule und das Ausfilterungs-Knotenmodul der Reihe nach über einen Bus oder eine Punkt-zu-Punkt-Datenübertragung in Reihe geschaltet werden, um eine geschlossene, ringförmige Struktur zu bilden,
wobei jedes der Knotenmodule die an demselben Knotenmodul befindliche Information cacht, um diese einem Funktionsmodul zur Konformitätsüberprüfung zur Verfügung zu stellen, und ferner auch die an demselben Knotenmodul befindliche Information unmittelbar an das nächste Knotenmodul übermittelt,
wobei jedes der Knotenmodule eine von demselben Knotenmodul stammende Information oder eine von dem vorhergehenden Knotenmodul stammende, abgelaufene Information feststellt und ggf. löscht, wobei eine Weiterleitung gelöschter Information untersagt wird,
wobei jedes der Knotenmodule eine Zeit-Feststellung für eine von demselben Knotenmodul stammende Information oder eine von dem vorhergehenden Knotenmodul stammende Information durchführt, innerhalb eines Konformitäts-Zeitfensters ein konformes Ereignis erfasst und dann die Information des konformen Ereignisses markiert,
wobei jedes der Knotenmodule die Information eines konformen Ereignisses oder die aus dem vorhergehenden Knotenmodul stammende, nicht abgelaufene Information eines separaten Ereignisses an das nächste Knotenmodule weiterleitet,
und wobei das Ausfilterungs-Knotenmodul eine Ereignisinformation mit einer Konformitäts-Markierung ausfiltert, die Weiterleitung der Ereignisinformation mit einer Konformitäts-Markierung an das nächste Knotenmodul unterbindet und die ausgefilterte Information konformen Ereignisses an einen Computer zur Datenerfassung übermittelt.

## Revendications

1. Procédé de réalisation de structure topologique annulaire de système d'imagerie TEP, **caractérisé en ce que** : la détermination de conformité des événements du système TEP est effectuée par une série de modules de confirmation distribués, ces modules de confirmation sont raccordés en série pour former une structure annulaire, chaque module de confirmation est un module nodal du lien ; la structure annulaire comprend au moins un module nodal de filtrage afin d'extraire les événements conformes.

2. Procédé selon la revendication 1, **caractérisé en ce que** : une pluralité de groupes de signaux électroniques de détecteurs converge vers un module de traitement d'événements, qui sélectionne un événement de pic à pleine énergie en effectuant la formation, la numérisation et l'analyse numérique des signaux ; une pluralité d'informations des modules de traitement d'événements est émise et converge vers un module nodal, la détermination de conformité est effectuée dans le module nodal.

3. Procédé selon la revendication 2, **caractérisé en ce que** : un système d'imagerie TEP comprenant en outre une pluralité de modules de conformité formant une structure annulaire, les modules de conformité de chacune desdites branches forment une branche étoilée via une pluralité de module de traitement.

4. Procédé selon la revendication 2, **caractérisé en ce que** : un système d'imagerie TEP comprenant en outre une pluralité de modules de conformité formant une structure annulaire, chacun desdits modules nodaux est doté des fonctions des modules de traitement des événements et des modules de conformité.

5. Une structure topologique annulaire de système d'imagerie TEP, **caractérisée en ce que** : elle comprend un module de traitement des événements destiné à recevoir et traiter les signaux multiplexes des détecteurs de rayons, des modules nodaux de détection de conformité recevant les événements des modules de traitement d'évènements multiplexes, et au moins un module nodal de filtrage des événements de conformité ;
Chacun desdits modules nodaux et modules nodaux de filtrage transmet en série les données du bus ou données point à point pour former une structure annulaire fermée.
Chaque module nodal place ses informations en cache afin de les utiliser sur le module fonctionnel de détection de conformité, et envoie directement ses informations au prochain module nodal ;
Chaque module nodal juge et supprime ses propres informations ou les informations obsolètes provenant du module nodal précédent, et interdit la poursuite de transmission des informations déjà supprimées ;
Chaque module nodal détermine le temps des informations provenant du module nodal précédent ou de ses propres informations, et détermine la conformité de l'événement dans la fenêtre temporelle conforme, puis marque les informations conformes.
Chaque module nodal transmet au prochain module nodal les informations des événements conformes ou les informations non obsolètes d'un simple événement provenant du module nodal précédent ;
Ledit module nodal de filtrage détecte les informations des événements marqués conformes, interdit la poursuite de la transmission des événements marqués conformes au prochain module nodal, et transmet les informations des événements conformes à l'ordinateur collectant les données.
